Europäisches Patentamt

European Patent Office

Office européen des brevets

(11) Publication number: **0 139 286 B1**

# EUROPEAN PATENT SPECIFICATION

(45) Date of publication of patent specification: **21.08.91**

(51) Int. Cl.⁵: **A61K 9/00, A61K 9/22, A61K 47/00, A61M 37/00**

(21) Application number: **84112310.2**

(22) Date of filing: **12.10.84**

The file contains technical information submitted after the application was filed and not included in this specification

(54) Prolonged sustained-release preparations.

(30) Priority: 14.10.83 JP 193064/83
20.10.83 JP 197181/83
01.11.83 JP 206226/83
14.12.83 JP 236994/83
14.12.83 JP 236995/83
14.12.83 JP 236996/83

(43) Date of publication of application:
**02.05.85 Bulletin 85/18**

(45) Publication of the grant of the patent:
**21.08.91 Bulletin 91/34**

(84) Designated Contracting States:
**CH DE FR GB LI SE**

(56) References cited:
**FR-A- 2 520 229**
**GB-A- 2 091 554**
**US-A- 3 016 895**
**US-A- 3 857 932**
**US-A- 4 245 635**

(73) Proprietor: **SUMITOMO PHARMACEUTICALS COMPANY, LIMITED**
**2-8, Dosho-machi 2-chome, Chuo-ku Osaka-shi Osaka-fu(JP)**

(72) Inventor: **Yamahira, Yoshiya**
**4-6-7-306, Tanaka-cho Higashinada-ku Kobe-shi Hyogo-ken(JP)**
Inventor: **Fujioka, Keiji**
**2-1, Kuwata-cho Ibaraki-shi Osaka-fu(JP)**
Inventor: **Sato, Shigeji**
**2-29-7, Oike Ibaraki-shi Osaka-fu(JP)**
Inventor: **Yoshida, Noboru**
**1-5-3-618, Higashi-Awaji Higashi-Yodogawa-ku Osaka-shi Osaka-fu(JP)**

(74) Representative: **Vossius & Partner Siebertstrasse 4 P.O. Box 86 07 67 W-8000 München 86(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to prolonged sustained release preparations. More particularly, it relates to prolonged sustained-release preparations in the form of bar-like or needle-like shaped preparations suitable for injection or injection-like administration, which comprise as an active ingredient a prostaglandin, prostacyclin, biohormone, interferon, interleukins or tumor necrosis factor in admixture with one or more of pharmaceutically acceptable biodegradable carriers which can be implanted into the body.

It is known that a medicament can be prepared in such a form that the medicament is embraced within a polymer, for example a polyethylene glycol diacrylate polymer, and is implanted into the body in order to sustain the release of the medicament. However, such a technique has various drawbacks in that the polymer is not biodegradable and hence it must be removed somehow after administration and in that the implantation must be done by operation with troublesome treatment. Nevertheless, it has been desired to create a sustained-release preparation for many medicaments.

It is the object of the present invention to provide an improved sustained-release preparation for prostaglandins, prostacyclins, biohormones, interferons, interleukins or tumor necrosis factors.

Another object of the invention is to provide a long (i.e. prolonged) sustained-release preparation in the form of bar-like or needle-like shaped preparations which can be injected or implanted into the body and can release the active ingredient and can maintain the desired level of the active ingredient in blood or in lesional region for a long period of time. A further object of the invention is to provide a device for administering a sustained-release preparation in the form of a needle-like or bar-like shape. These and other objects and advantages of the present invention will be apparent to persons skilled in the art from the following description.

These objects have been achieved by the surprising finding that the desired sustained-release preparation can be obtained by admixing the active ingredient with a specific biodegradable carrier and that the formed product of the preparation in the form of a bar-like or needle-like shape is very useful for injection or for implanting into the body and shows excellent effects with respect to release-sustaining of the active ingredient.

The sustained-release preparations of the present invention are in the form of a bar-like or needle-like shaped preparation, which comprises (i) a prostaglandin, prostacyclin, biohormone, interferon, interleukins or a tumor necrosis factor in admixture with (ii) one or more pharmaceutically acceptable biodegradable carriers which can be absorbed or be subject to enzymolysis in the body and can be implanted within the body, such as proteins (e.g. collagen, gelatin, albumin), polysaccharides (e.g. chitin), or high molecular compounds (e.g. polylactic acid, polyglutamic acid). The sustained release preparation is formed in a bar-like or needle-like shape and can be injected or implanted into the body.

The active ingredient used in the present invention includes also 4-carbamoyl-imidazolium-5-oleate (other name: 4-carbamoyl-5-hydroxy-imidazole or SM-108) or a salt or a hydrate thereof, known as a new antitumor agent and effective as a metabolic antagonist in purine de novo synthesis.

Interferons, interleukins, and tumor necrosis factors are somewhat different to each other, but are common in that they have very similar molecular weights and are glycoproteins or proteins and have similar pharmacological and physico-chemical properties as $\alpha$-interferon as will be shown in experiments hereinafter. Prostaglandins and prostacyclines are different to each other, but they have in common that they have similar molecular weights as SM-108, as will be shown in experiments hereinafter. All of these compounds are prepared for the desired excellent sustained-release preparation of the present invention.

The biodegradable carrier used in the present invention means a carrier which can easily be absorbed or be subject to enzymolysis in the body and can be implanted into the body. Suitable examples of the biodegradable carrier are proteins such as collagen, gelatin, albumin; polysaccharides such as chitins; and synthetic high molecular compounds such as polyglycolic acid, polylactic acid, polyglutamic acid, or the like. These substances can be used alone or in any combination of two or more thereof, but for reasons of safety and easy handling, collagen or gelatin or a mixture thereof are preferable. Collagen is a main protein of the connective tissue of animals and has less antigenicity, and hence, has widely been used as a safe operation yarn in various medical operations. The collagen may be an atelocollagen having far less antigenicity which is obtained by removing the telopeptide region by treating collagen with an enzyme (e.g. pepsin) in order to make it safer. Gelatin is a protein derived from collagen. Gelatin is a high molecular weight amphoteric electrolyte which has less antigenicity and convertibility properties between sol and gel forms and is low in costs, and hence it has already been accepted as a safe substance for medical use.

The preparation of the present invention contains the active ingredient in an amount of in which the active ingredient is usually used. For example, interferon is usually contained in an amount of $10^4$ to $10^9$ IU, preferably $10^5$ to $5 \times 10^8$ IU per dosage

unit, and SM-108 or a salt or hydrate thereof is usually contained in an amount of 1 mg to 2 g, preferably 10 mg to 1 g per dosage unit.

Besides, the ratio of the medicament and the carrier is not specified, but for example, interferon is preferably incorporated in an amount of $10^3$ to $10^8$ IU per 1 mg of the carrier, and SM-108 is preferably incorporated in an amount of 0.01 to 1 mg per 1 mg of the carrier.

The sustained-release preparation of the present invention can be prepared by the following method.

The active ingredient or an aqueous solution thereof is mixed with a biodegradable carrier or an aqueous solution thereof, and the mixture is homogeneously mixed by stirring while preventing occurrence of foam as much as possible. The resulting mixture is optionally concentrated at a low temperature and further optionally spray-dried or lyophilized. In the preparation, there may optionally be incorporated conventional pharmaceutically acceptable additives such as stabilizers, preservatives, local anesthetic agents, and some agents for aiding formability into special shapes of preparations or for release-sustaining of the active ingredient. These additives are not specified, but suitable examples of the agents for aiding formability are methylcellulose, ethylcellulose, hydroxypropylcellulose, polyvinylpyrrolidone, glycolic acid-lactic acid copolymer, polyethylene glycol, propylene glycol, ethyl alcohol, or the like. Suitable examples of the agents for aiding release-sustaining of the active ingredient are cellulose acetate phthalate, ethylcellulose, methylcellulose, hydroxypropylmethylcellulose, calcium phosphate, lactic acidglycolic acid copolymer, corn starch, rice starch, potato starch, cellulose, arginates, or the like.

The preparation thus obtained is pulverized to powders under cooling with dry ice or liquid nitrogen, or by any other conventional pulverization method. The powder is compressed together with other additives if specific shapes should be formed, such as a needle-like or fine bar-like shaped preparations (diameter: about 0.5 mm - 1.5 mm, length: about 5 mm - 15 mm), which can be inserted into a body with a forceps needle for fiberscope, an indwelling needle, or other appropriate administration device as mentioned hereinafter. Alternatively, the powdery preparation is previously entered into a mold, followed by concentrating at a low temperature or by lyophilizing to compress and form a needle-like or a fine bar-like shaped preparation.

All steps for preparing the desired sustained-release preparations are carried out under sterilized conditions because the preparations are used as an injection or for implanting into a body.

The long sustained-release preparation of the present invention can be administered to the patients by various methods, for example, by inserting a fine tube into the body at the desired region with an appropriate means, such as a catheter and then inserting the needle-like shaped preparation of the present invention by passing through inside the fine tube, or by inserting the preparation of the present invention directly into the body at the lesional region by means of forceps needles of a fiberscope.

The present invention provides also an improved device for administering the needle-like or bar-like shaped preparations of the present invention.

The device for administration of the sustained-release preparation and the mode of their administration are explained referring to the accompanying drawings.

Fig. 1 shows an embodiment of a device for administering the preparation of the present invention which comprises a fine tube and an inner needle.

Fig. 2 is an embodiment showing a state where the device as shown in Fig. 1 is stabbed into the body.

Fig. 3 is an embodiment showing a state where a needle-like shaped solid preparation is administered into the body by the device as shown in Fig. 1.

Fig. 4 shows an embodiment of a needle for administering a needle-like shaped solid preparation of the present invention wherein the solid preparation is held in the needle.

Fig. 5 is an embodiment showing a state where the needle-like shaped solid preparation is administered into the body with the administration needle as shown in Fig. 4.

Fig. 6 is an embodiment of a needle for administering a needle-like shaped solid preparation of the present invention which is provided with a removable cover for preventing the solid preparation contained therein from falling down.

Fig. 7 is a graph showing the relation between the blood level of an active ingredient and the time elapsed after intramuscular administration of the preparation in rabbits.

Fig. 8 is a graph showing the rate of releasing of the active ingredient from the needle-like shaped sustained-release preparation of the present invention.

The device for administering a needle-like shaped preparation comprises (i) a fine tube and (ii) an inner needle which can freely slide within the fine tube.

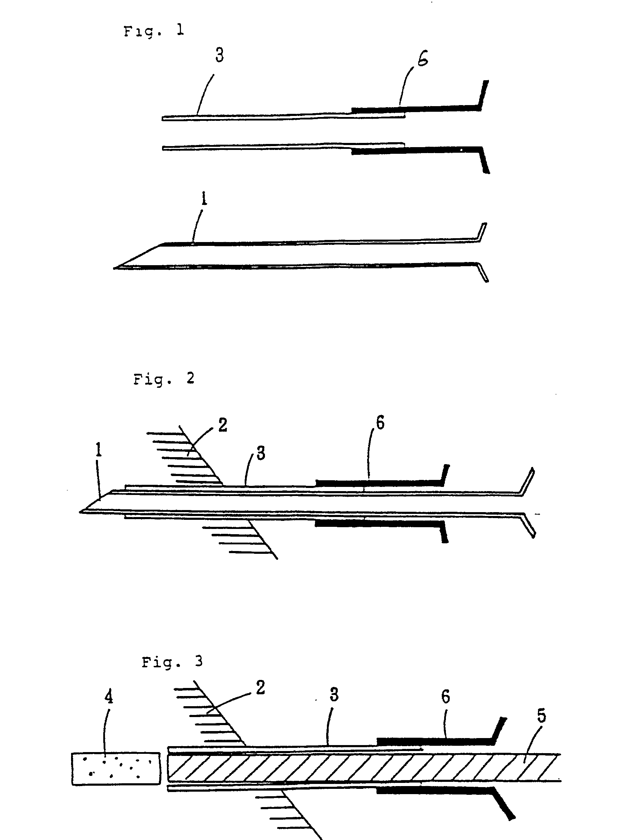

The fine tube in the above device is a tube having an inner diameter of 0.5 to 5 mm which can be inserted partly into a body. The length of the tube is not specified but may be any size convenient for injection, and is usually in the range of 2 cm to 10 cm. The material for preparing the tube may be any kind of material compatible with the body. The inner needle has a sharp tip as shown in the accompanying Fig. 1 and has an outer diameter as the same as or smaller than the inner diameter of the above tube.

The device for administering a needle-like shaped preparation is usually used with being held in a holding device (6), but may be used with being held at the tip of forceps needle of fiberscope.

The administration manner of the needle-like shaped preparation with the above device is explained in more detail below. Firstly, the inner needle (1) is stabbed into a portion of the body (2) and simultaneously the fine tube (3) is inserted into the body by sliding its inner wall along the outer wall of the inner needle (1) (cf. the accompanying Fig. 2). Thereafter, the inner needle (1) is pulling off, and then the needle-like shaped preparation (4) is inserted into the body by passing through the inner slit of the fine tube (3), and finally the fine tube is taken off. The insertion of the needle-like shaped preparation into the body is usually carried out by inserting the preparation into the fine tube after pulling off of the inner needle (1), pushing the preparation with a pushing pole (5) till the inside of the body (2) (cf. the accompanying Fig. 3). The pushing pole (5) may be any pole which can be inserted into and can freely slide inside the fine tube (3), and the above inner needle (1) may also be used as the pushing pole.

In order to insert the preparation of the present invention into a deep region of the body, i.e. internal organs such as stomach wall, the device for fiberscope may be used, and by easily handling the fiberscope, one can effect procedures such as stabbing of the inner needle, insertion of the fine tube, pulling off of the inner needle, administration of the preparation and taking off of the fine tube.

The preparation which can be administered by the above device may be any one of needle-like or bar-like shaped preparations which can be inserted and held within the fine tube (3).

Alternative devices for insertion of the needle-like shaped solid preparations are injection needles provided with a inner pushing pole which can smoothly slide within the slit of the needle. The injection needles include conventional injection needles. The pushing pole has an outer diameter which is the same or smaller as the inner diameter of the injection needle and it can push a needle-like shaped solid preparation having a diameter of 0.5 to 5 mm.

The device for insertion of a needle-like shaped solid preparation may be held with a conventional holding device (7), but it may be also used by holding it at the tip of forceps needle for fiberscope.

The administration manner of the needle-like shaped solid preparation of the present invention is explained in more detail below.

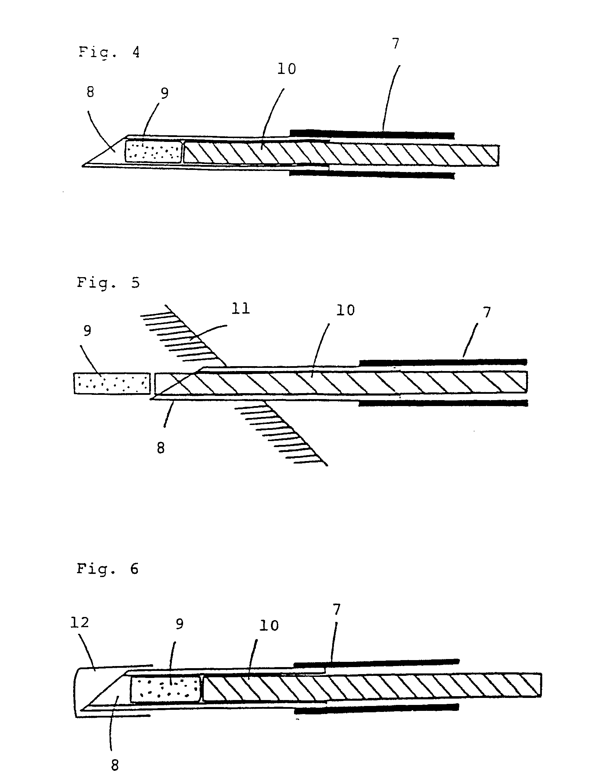

Previously, the needle-like shaped solid preparation (9) is held within the injection needle (8) (cf. the accompanying Fig. 4), and the injection needle (8) is stabbed at the portion of the body (11) and simultaneously the preparation is administered into the body (11) by pushing it with a pushing pole (10) (cf. the accompanying Fig. 5). In order to administer the preparation to deeper regions of the body such as the stomach wall, it may be administered with a fiberscope as mentioned above. In such a case, for preventing of falling down of the preparation from the needle, it is preferable to provide a removable cover (12) at the tip of the needle (cf. the accompanying Fig. 6). The solid preparation useful for the above administration may be in any form such as needle-like or bar-like shape which can be held in a conventional injection needle.

As is explained above, according to the device for injection of the present invention, the preparation of the present invention can easily be administered, for example, by insertion into the internal organs (with a fiberscope) and for administerating systematically or topically at the body surface (with a device or needle as mentioned above). These methods are practically and clinically very useful, and it is a novel idea that a biodegradable solid preparation is administered in the above-mentioned manner.

The present invention is illustrated by the following Experiments and Examples, but should not be construed to be limited thereto.

Experiment 1

There were used as the test samples a needle-shaped preparation of $\alpha$-interferon-collagen prepared in Example 1 disclosed hereinafter (Sample A) and a reference (an aqueous injection of $\alpha$-interferon originated from Namalwa cells). The test samples were each administered intramuscularly to rabbit, and the change of level in blood of the active ingredient with lapse of time was measured by RIA (radioimmunoassay) method. Two rabbits were used for each sample, and the test samples were each administered in a dose of $10^6$ U/kg. The measurement of blood levels was performed in average in two rabbits.

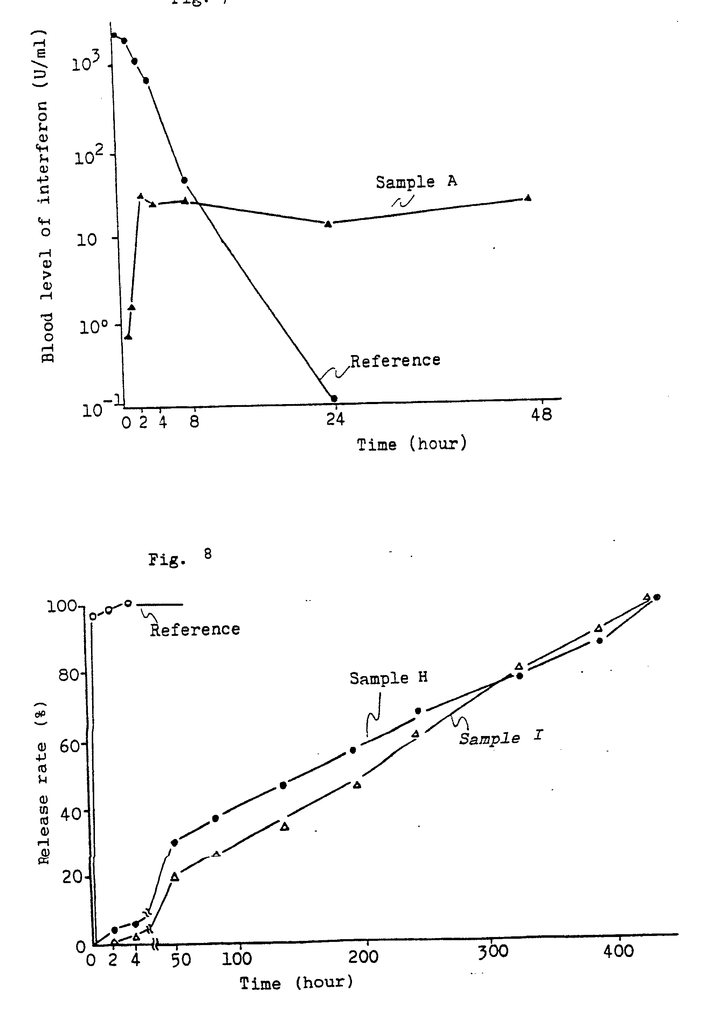

The results are shown in the accompanying Fig. 7. In Fig. 7, ▲ is the graph of Sample A, and ● is that of reference ($\alpha$-interferon aqueous injection). As is clear from the figure, sample A showed release-sustaining, and even after 48 hours, a blood level of several tens unit/ml was maintained.

Thus, it is also suggested by the test of in vivo using rabbits that the preparation of the present invention is useful clinically.

Experiment 2

In order to test the release rate of indomethacin from the sustained-release preparation of the present invention (formed in a needle shape), the preparations of Examples 8 and 9 disclosed hereinafter (Samples H and I) and a reference (indomethacin alone) were subjected to a release test by a rotatory basket method with a basket stirring element as defined in U.S. Pharmacopeia.

The results are shown in the accompanying Fig. 8. In Fig. 8, ● is a graph of Sample H, △ is that of Sample I, and ○ is that of reference (indomethacin alone). As is clear from the figure, in case of indomethacin alone, it released immediately, but on the other hand, in case of the needle-shaped sustained-release preparation of the present invention, the release continued for more than 10 days.

Example 1

An aqueous solution of α-interferon (titer: 4.9 MU/ml) (100 ml) and 2 % atelocollagen (50 g) are homogeneously mixed while stirring and preventing occurrence of foam as much as possible. The mixture is lyophilized and pulverized at a low temperature using liquid nitrogen. The pulverized product thus obtained is formed under compression to give a needle-shaped sustained-release preparation (A) wherein interferon is contained in an amount of 10 MU per 1 needle.

Example 2

An aqueous solution of α-interferon (titer, 4.9 MU/ml) (100 ml), 2 % atelocollagen (50 g), human serum albumin (150 mg) and thimerosal (120 μg) are homogeneously mixed with preventing occurrence of foam as small as possible. The mixture is lyophilized and pulverized at a low temperature using liquid nitrogen. The pulverized product thus obtained is subjected to a compression molding to give a needle-like shaped sustained-release preparation (Sample B) wherein interferon is contained in an amount of 10 MU per 1 needle.

Example 3

An aqueous solution of α-interferon (titer, 4.9 MU/ml) (100 ml) and 2 % collagen (50 g) are homogeneously mixed while preventing occurrence of foam as much as possible. The mixture is lyophilized and pulverized at a low temperature using liquid nitrogen. The pulverized product thus obtained is subjected to a compression molding to give a bar-like shaped sustained-release preparation (Sample C) wherein interferon is contained in an amount of 5 MU per 1 bar.

Example 4

An aqueous solution of α-interferon (titer, 4.9 MU/ml) (100 ml) and atelocollagen powder (1 g) are mixed and the mixture is dissolved by adding thereto 0.1 N hydrochloric acid, and the resulting solution is entered into a mold and lyophilized. The lyophilized product is formed under compression to give a needle-shaped sustained-release preparation (Sample D) wherein interferon is contained in an amount of 10 MU per 1 needle.

Example 5

An aqueous solution of α-interferon (titer, 4.9 MU/ml) (100 ml) and gelatin (1 g) are homogeneously mixed at 60°C while preventing occurrence of foam as much as possible. The mixture is lyophilized and pulverized at a low temperature using liquid nitrogen. The pulverized product thus obtained is subjected to a compression molding to give a needle-like shaped sustained-release preparation (Sample E) wherein interferon is contained in an amount of 5 MU per 1 needle.

Example 6

An aqueous solution of α-interferon (titer, 4.9 MU/ml) (100 ml) and atelocollagen powder (1 g) are mixed and the mixture is dissolved by adding thereto 0.1 N hydrochloric acid, and the resulting solution is lyophilized and pulverized at a low temperature using liquid nitrogen. To the pulverized product is added methylcellulose (25 % by weight based on the weight of the pulverized product), and thereto is added distilled water for injection so that the solid content becomes 20 % by weight. The mixture is kneaded. The kneaded product is entered into a mold, lyophilized, and then, compressed to give a needle-shaped sustained-release preparation (Sample F) wherein interferon is contained in an amount of 10 MU per 1 needle.

Example 7

An aqueous solution of α-interferon (titer, 4.9 MU/ml) (100 ml), 2 % atelocollagen (50 g) and tespamin (triethylenethiophosphoramide, which is known as an antineoplastic) (98 mg) are homogeneously mixed while preventing occurrence of foam as much as possible. The mixture is lyophilized and pulverized at a low temperature using liquid nitrogen. The pulverized product thus obtained is subjected to compression molding to give a needle-shaped sustained-release preparation (Sample G) wherein interferon and tespamin are contained in an amount of 10 MU and about 2 mg per 1 needle, respectively.

## Example 8

To a 1 % aqueous solution of atelocollagen (200 g) is added indomethacin (4 g), and the mixture is concentrated to about 20 ml. The mixture is poured into a silicone tube and frozen. The tube is cut, and the freezed product is crosslinked with glutaraldehyde in a gaseous phase for 4 days to give a needle-shaped sustained-release preparation (Sample H).

## Example 9

To a 1 % aqueous solution of atelocollagen (200 g) is added indomethacin (4 g), and the mixture is concentrated to about 20 ml. The mixture is poured into a silicone tube and frozen. The tube is cut, and the freezed product is dried. The resulting product is inserted into a larger-size silicone tube wherein 40 % atelocollagen is contained, and is frozen. The frozen product is taken out from the tube and then is crosslinked with glutaraldehyde in a gaseous phase for 4 days to give a double structure, needle-shaped sustained-release preparation (Sample I).

## Example 10

Powdery collagen (2 g) is swollen with a small amount of distilled water and is dissolved by adding thereto 0.1N-HCl. To the solution are added SM-108 hydrate (2 g) and sodium hydrogensulfite (100 mg), and is further added distilled water so that the collagen concentration becomes 2 % by weight. The mixture is entered into a mold, lyophilized and then subjected to compression molding to give a needle-shaped sustained-release preparation (Sample J).

## Example 11

The needle-shaped preparation obtained in the same manner as described in Example 10 is inserted into a larger-size silicone tube wherein 40 % atelocollagen is contained, and frozen. The frozen product is taken out and then crosslinked with glutaraldehyde in gaseous phase for 4 days to give a needle-shaped sustained-release preparation having double structure (Sample K).

**Claims**

1. An injectable sustained-release preparation which comprises a prostaglandin, prostacyclin, biohormone, interferon, interleukin or tumor necrosis factor as an active ingredient and a pharmaceutically acceptable proteinaceous biodegradable carrier in the form of a needle-like or bar-like shape.

2. An injectable sustained-release preparation which comprises a prostaglandin, prostacyclin, biohormone, interferon, interleukin or tumor necrosis factor as an active ingredient and a pharmaceutically acceptable proteinaceous biodegradable carrier in the form of a needle-like or bar-like shape obtainable by mixing the active ingredient and the carrier or an aqueous solution thereof and forming the mixture into a needle-like or bar-like shape.

3. The preparation according to claim 1 or 2, wherein the mixture is spray-dried or lyophilized before it is formed.

4. The preparation according to claim 3, wherein the mixture is lyophilized.

5. The preparation according to any one of claims 1 to 4, wherein the forming is carried out by compressing a powder of the carrier matrix.

6. The preparation according to any one of claims 1 to 4, wherein the forming is carried out by pouring the resulting mixture into a mold.

7. The preparation according to claim 1 or 2, wherein the active ingredient is a biohormone, interferon, interleukin or tumor necrosis factor.

8. The preparation according to claim 1 or 2, wherein the biodegradable carrier is collagen, atelocollagen, gelatin or a mixture thereof.

9. The preparation according to claim 1 or 2, wherein the biodegradable carrier in collagen, atelocollagen, or a mixture thereof.

10. The preparation according to claim 1 or 2, wherein the active ingredient is interferon and the biodegradable carrier is collagen, atelocollagen, or a mixture thereof.

11. Method for producing an injectable sustained-release preparation according to any of claims 2 to 10.

**Revendications**

1. Préparation injectable à libération prolongée oui comprend une prostaglandine, une prostacycline, une hormone biologique, un interféron, une interleukine ou un facteur nécrosant des tumeurs comme ingrédient actif et un véhicule biodégradable protéinique pharmaceutiquement acceptable sous forme d'une aiguille ou d'un barreau.

2. Préparation injectable à libération prolongée qui comprend une prostaglandine, une prostacycline, une hormone biologique, un interféron, une interleukine ou un facteur nécrosant des tumeurs comme ingrédient actif et un véhicule biodégradable protéinique pharmaceutiquement acceptable, sous forme d'une aiguille ou d'un barreau, qui peut être obtenue par mélange de l'ingrédient actif et du véhicule, ou d'une solution aqueuse correspondante, et façonnage du mélange en une aiguille ou en un barreau.

3. Préparation selon la revendication 1 ou 2, dans laquelle le mélange est séché par pulvérisation ou lyophilisé avant d'être façonné.

4. Préparation selon la revendication 3, dans laquelle le mélange est lyophilisé.

5. Préparation selon l'une quelconque des revendications 1 à 4, dans laquelle le façonnage est effectué par compression d'une poudre de la matrice de véhicule.

6. Préparation selon l'une quelconque des revendications 1 à 4, dans laquelle le façonnage est effectué par coulée du mélange obtenu dans un moule.

7. Préparation selon la revendication 1 ou 2, dans laquelle l'ingrédient actif est une hormone biologique, un interféron, une interleukine ou un facteur nécrosant des tumeurs.

8. Préparation selon la revendication 1 ou 2, dans laquelle le véhicule biodégradable est le collagène, l'atélocollagène, la gélatine ou un de leurs mélanges.

9. Préparation selon la revendication 1 ou 2, dans laquelle le véhicule biodégradable est le collagène, l'atélocollagène ou un de leurs mélanges.

10. Préparation selon la revendication 1 ou 2, dans laquelle l'ingrédient actif est un interféron et le véhicule biodégradable est le collagène, l'atélocollagène ou un de leurs mélanges.

11. Procédé pour préparer une préparation à libération prolongée injectable selon l'une quelconque des revendications 2 à 10.

**Patentansprüche**

1. Ein injizierbares langzeitwirkendes Mittel, umfassend ein Prostaglandin, Prostacyclin, Biohormon, Interferon, Interleukin oder einen Tumornekrosefaktor als Wirkstoff und einen pharmazeutisch verträglichen, eiweißartigen und biologisch abbaubaren Träger mit einer nadelförmigen oder stäbchenförmigen Gestalt.

2. Ein injizierbares langzeitwirkendes Mittel, umfassend ein Prostaglandin, Prostacyclin, Biohormon, Interferon, Interleukin oder einen Tumornekrosefaktor als Wirkstoff und einen pharmazeutisch verträglichen, eiweißartigen und biologisch abbaubaren Träger mit einer nadelförmigen oder stäbchenförmigen Gestalt, erhältlich durch Vermischen des Wirkstoffs mit dem Träger oder einer wäßrigen Lösung davon und Formen des Gemisches zu einer nadelförmigen oder stäbchenförmigen Gestalt.

3. Mittel nach Anspruch 1 oder 2, wobei das Gemisch vor dem Formen spraygetrocknet oder gefriergetrocknet wird.

4. Mittel nach Anspruch 3, wobei das Gemisch gefriergetrocknet wird.

5. Mittel nach einem der Ansprüche 1 bis 4, wobei das Formen durch Verpressen eines Pulvers des Trägergrundstoffs durchgeführt wird.

6. Mittel nach einem der Ansprüche 1 bis 4, wobei das Formen durch Eingießen des entstandenen Gemisches in eine Gußform durchgeführt wird.

7. Mittel nach Anspruch 1 oder 2, in dem der Wirkstoff ein Biohormon, Interferon, Interleukin oder Tumornekrosefaktor ist.

8. Mittel nach Anspruch 1 oder 2, in dem der biologisch abbaubare Träger Collagen, Atelocollagen, Gelatine oder ein Gemisch daraus ist.

9. Mittel nach Anspruch 1 oder 2, in dem der biologisch abbaubare Träger Collagen, Atelocollagen oder ein Gemisch daraus ist.

10. Mittel nach Anspruch 1 oder 2, in dem der Wirkstoff Interferon und der biologisch abbaubare Träger Collagen, Atelocollagen, oder ein Gemisch daraus ist.

11. Verfahren zur Herstellung eines injizierbaren langzeitwirkenden Mittels nach einem der Ansprüche 2 bis 10.

Fig. 1

Fig. 2

Fig. 3

Fig. 4

Fig. 5

Fig. 6

EP 0 139 286 B1

Fig. 7

Fig. 8

11